(19) Europäisches Patentamt
European Patent Office
Office européen des brevets

(11) Publication number: **0 135 890 A2**

# (12) EUROPEAN PATENT APPLICATION

(21) Application number: 84110894.7

(22) Date of filing: 12.09.84

(51) Int. Cl.⁴: **A 61 F 13/04**

(30) Priority: 13.09.83 US 532352

(43) Date of publication of application: 03.04.85 Bulletin 85/14

(84) Designated Contracting States: DE FR GB NL SE

(71) Applicant: ISOPEDIX CORPORATION, 17662 Armstrong Avenue, Irvine California 92714 (US)

(72) Inventor: Honeycutt, Travis W., 8 Lago Süd, Irvine California 92715 (US)

(74) Representative: Reinländer & Bernhardt Patentanwälte, Orthstrasse 12, D-8000 München 60 (DE)

## (54) Hardenable fabric.

(57) A method of producing a fabric which is initially uniformly flexible, but is resultantly substantially selectively hardened without significant dimensional shrinkage comprises providing a first fiber capable of shringking and substantially hardening upon the exposure of said first fiber to heat. Thereafter, the first fiber is woven or knitted with a second fiber, said second fiber being an elastomer which is knitted with said first fiber while being substantially elongated and thus under tension. The knitting or weaving operation is completed at which time the tension imposed upon the elastomer is substantially lessened or removed. A portion of the fabric is selectively heated to substantially harden selective areas and an immobilizing agent is applied in at least a portion of those selective areas.

EP 0 135 890 A2

0135890

Description

Hardenable Fabric

## Technical Field of Invention

The present method deals with producing a fabric which is flexible but capable of being substantially hardened through the application of heat and an immobilizing agent. The fabric itself is capable of being configured into a number of divergent shapes for various uses, however, the fabric is particularly well-adapted for being configured into the shape of a tube for use as an orthosis.

## Background Art

Noting the disadvantages inherent in the use of plaster of paris casts as well as other prior art cast materials, prior application No. 84 106 882.8 disclose for the first time a fabric which is flexible but capable of being substantially hardened through the application of heat. The fabric was thought to be uniquely adapted as a substitute for prior cast materials by exhibiting rigidity but not the secondary characteristics of prior art cast materials, which have resulted in patient discomfort and even physical disorders.

As the above referenced parent applications fully disclose, a hardenable fabric could be provided for use in a multitude of applications. Its primary advantage resides in the use of the fabric as an orthosis for the fabric is capable of being substantially hardened

without substantial shrinkage. When used as an orthosis, the fabric exhibits a number of improvements over prior art materials. More specifically, an orthosis fabricated from applicant's hardenable fabric is radiolucent, i.e., substantially transparent to x-ray penetration, light weight and breathable. Further, an orthopedic practitioner could apply and remove a cast made from such fabric with a degree of ease and convenience not before possible.

Although the hardenable fabric continues to represent a substantial improvement over corresponding materials of the prior art, it has been determined that, even after heating the fabric and thus hardening the same, there remains some interstitial movement between strands of a woven or knitted fibers which, under certain limited circumstances, might be thought objectionable by practitioners. Generally, some limited movement is desired in the ideal orthosis for controlled movement of the restricted limb can help to prevent atrophying and promote healing. There is always some dimensional spacing between the injured limb and the interior wall of the standard plaster of paris cast but this allows fulcrums to develop, causing undesirable bending moments in portions of the limb. The ideal orthosis allows movement of the total restricted limb but embraces the limb and does not permit dimensional spacing. Nevertheless, "rock" hardness of an orthosis is desirable in certain limited applications.

It is thus an object of the present invention to provide a fabric which is selectively hardenable and which does not exhibit the interstitial movement of applicant's prior hardenable fabrics.

It is yet a further object of the present invention to provide for a selectively hardenable fabric which is capable of being configured into an

orthosis which does not exhibit interstitial movement which appears in applicant's prior hardenable fabric and yet which exhibits a number of the physical advantages inherent in such a fabric which are not present in prior rigid orthoses.

Summary of Invention

The present invention deals with a fabric and method of producing the fabric which is flexible, but which is capable of being substantially selectively hardened through the application of heat and an immobilizing agent without exhibiting significant dimensional shrinkage. The method includes providing a first fiber which, prior to its being incorporated with a second fiber, is impressed with a memory so that upon the application of heat, the fiber is inclined to become shorter in length and significantly harder and less pliable.

Once the first fiber has been preconditioned, it is combined with a second fiber by, for example, knitting or weaving. Although virtually any appropriate weaving or knitting processes are contemplated for combining the fibers, it is basic to the present invention that the second fiber, which is preferably an elastomer, is combined with the first fiber while being substantially elongated and thus under tension. Once the knitting or weaving operation is completed, the tension imposed upon the elastomer is substantially lessened or removed.

Preferably in areas which have been hardened by heating, an immobilizing agent is applied which substantially reduces or completely eliminates all interstitial movement between the fibers in selective areas. By practicing this invention, one can achieve, in a single fabric, areas of flexibility, moderate hardness and "rock" hardness. Preferably, all of the

areas would remain radiolucent, light weight and breathable.

Detailed Description of the Invention

The basic fabric of the present invention can be configured from the processes as fully described in prior application No. 84 106 882.8

To summarize, the fabric can be prepared by providing a first fiber capable of shrinking and substantially hardening upon the exposure of said first fiber to heat. Preferably, the first fiber is a material capable of cross-linking which has been exposed to radiation to so cross-link a substantial fraction of the molecules of the fiber. The fiber can then be drawn to substantially increase its length at which time it is knitted or woven with a second fiber, the second fiber being an elastomer which is knitted or woven with the first fiber while being substantially elongated and thus under tension. The knitting or weaving operation is then completed at which time the tension imposed upon the elastomer is substantially lessened or removed.

As a further refinement upon this method, the first fiber can be wrapped around the elastomer as a single or double helix while the elastomer is, again, under tension. When the helix-forming operation is completed, the tension imposed upon the elastomer can be substantially lessened or removed at which time the composite is knitted or woven into a final fabric.

In yet another embodiment, after the single or double helix has been formed and the elongated elastomer allowed to somewhat relax, the composite can be knitted or woven with yet another elastomer which, in turn, is substantially elongated and under tension during the knitting or weaving operation. This latter embodiment results in a fabric which exhibits an

enhanced bulk or thickness in comparison to earlier embodiments referred to above.

As noted in the above-referenced parent applications, the first fiber is preferably a member selected from the group consisting of a polyolefin, a polyamide, a polyester, a polyacrylonitrile, a cellulosic or its derivatives and a polyimidazole. The elastomer is preferably a member selected from the group consisting of spandex and rubber.

In practice, the hardenable fabric can be substantially hardened in selective areas by exposing said areas to heat. It has been found that the heat generated from an ordinary portable hairdryer is sufficient to relax the molecular orientation of the first fiber thus reducing its length and increasing its hardness. Even when this is done, however, some interstitial movement exists between adjacent fibers of the fabric body which results in a slight "give" of the fabric even in hardened areas.

It has surprisingly been found that the fabric, in hardened areas, can be made "rock" hard by applying an immobilizing agent which, as the name implies, results in the immobilization of woven or knitted fiber interstices. There are a number of alternative formulations which can be employed as the immobilizing agent. For example, a 5% aqueous corn starch solution can be employed. In practice, the aqueous corn starch mixture is first boiled and allowed to cool to approximately 140°F. at which time it is merely applied to the surface of the fabric in heat hardened areas. In approximately 30 minutes, the fabric becomes "rock" hard in said areas.

As an alternative, one can apply gelatin and gelatin derivatives. The gelatin, for example Porcine gelatin, can be added to water in an approximately 5% concentration and the solution boiled and applied to

the hardenable fabric in selected areas at approximately 120°F. Similarly, one can employ a sulfonated polyester available from Eastman Kodak Company sold under the trademark Eastman Size WNS. The sulfonated polyester can be employed as an aqueous dispersion at a concentration of approximately 40%. When applied to selected areas of the hardenable fabric, those selected areas become extremely hard in approximately 30 to 60 minutes.

Aromatic diisocyanates can also be employed as immobilizing agents. Particularly, an overequivalence of 4,4-methyl diphenyl diisocyanate which has been partially polymerized with a polyol (poly-diol) can be employed. When so applied, the hardened areas can be oversprayed with water and a catalyst such as stannous octoate to catalyze the completion of the polymerization reaction.

As the preferred embodiment of the present invention, the selective areas of the hardenable fabric can be further hardened with α-cyanoacrylic acid esters of the following structure:

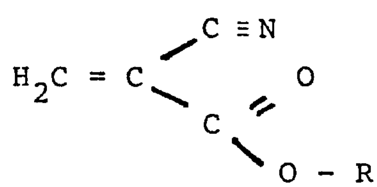

wherein R is a member selected from the group consisting of

$CH_3$-, $CH_3CH_2$-, $CH_3CH_2CH_2$-, $CH_3CH_3CH$- and aliphatic, aromatic or mixed aliphatic/aromatic substituents where $C \leq 17$. The α-cyanoacrylic acid esters represent a class of preferred immobilizing agents for a number of reasons. Firstly, they are convenient to use either at low viscosity to "penetrate" the fabric or at

relatively high viscosity to sit upon the surface of the fabric. As such, these materials should be used at a viscosity range of between approximately 5 to 1000 cps and, preferably, 50 to 500 cps and are available from Pacer Technology being sold under the trademarks PX50 and PX500. Secondly, the α-cyanoacrylic acid esters do not substantially reduce the breathability of the fabric. Although the previously disclosed substitutes for the α-cyanoacrylic acid esters can be employed by maintaining the breathability by virtue of the application of the immobilizing agents only in selective areas, the α-cyanoacrylic acid esters can be uniformly applied to the entire surface of the orthosis while substantial breathability of a fabric body is retained.

The polymerization of the α-cyanoacrylic acid esters can be catalyzed in several ways. The polymerization can take place without an externally applied catalyst, however, the process can be enhanced by providing a sensitizer in the fabric body during its manufacture. Sensitizers can ideally comprise an anionic or cationic detergent or surfactant. Alternatively, any alkaline generating material can be employed such as e.g. sodium bicarbonate. As illustrative, an amine, such as an aryl alkyl amine which is sold by Pacer Technology Inc. as a solution in trichlorotrifluoroethane. These catalytic agents, particularly the amines, can be merely oversprayed onto the hardenable fabric.

Claims

1. Method of producing a fabric which is initially uniformly flexible but is resultantly substantially selectively hardened without significant dimensional shrinkage comprising:

A) providing a first fiber capable of shrinking and substantially hardening upon the exposure of said first fiber to heat;

B) weaving or knitting said first fiber with a second fiber, said second fiber being an elastomer which is knitted or woven with said first fiber while being substantially elongated and thus under tension;

C) completing the knitting or weaving operation of step B at which time the tension imposed upon the elastomer is substantially lessened or removed;

D) selectively heating at least a portion of said fabric to substantially harden said selective areas; and

E) applying an immobilizing agent in at least a portion of said selective areas.

2. A method of producing a fabric which is initially uniformly flexible but is resultantly substantially selectively hardened without significant dimensional shrinkage comprising:

A. providing a first fiber capable of shrinking and substantially hardening upon the exposure of said first fiber to heat;

B) forming a helix of said first fiber around a second fiber, said second fiber being an elastomer which is combined with said first fiber while being substantially elongated and thus under tension;

C) completing the helix-forming operation of step B at which time the tension imposed upon the elastomer is substantially lessened or removed;

D) knitting or weaving a composite comprising said first and second fibers into a final fabric;

E) selectively heating at least a portion of said fabric to substantially harden said selective areas; and

F) applying an immobilizing agent in at least a portion of said selective areas.

0135890

3. A method of producing a fabric which is initially uniformly flexible but is resultantly substantially hardened without significant dimensional shrinkage comprising:

A) providing a first fiber capable of shrinking and substantially hardening upon the exposure of said first fiber to heat;

B) forming a helix of said first fiber around a second fiber, said second fiber being an elastomer which is combined with said first fiber while being substantially elongated and thus under tension;

C) completing the helix-forming operation of step B at which time the tension imposed upon the elastomer is substantially lessened or removed;

D) knitting or weaving the composite comprising said first and second fibers with a third fiber, said third fiber being another elastomer which is knitted or woven with said composite while being substantially elongated and thus under tension;

E) completing the knitting or weaving operation of step D at which time the tension imposed upon the third fiber is substantially lessened or removed;

F) selectively heating at least a portion of said fabric to substantially harden said selective areas; and

G) applying an immobilizing agent in at least a portion of said selective areas.

4. The method of claims 1, 2 or 3 wherein the fabric is in the shape of a tube.

5. The method of calims 1, 2 or 3 wherein the tube is configures for use as an orthosis.

6. The method of claims 1, 2 or 3 wherein said first fiber is a member selected from a group consisting of a polyolefin, a polyamide, a polyester, a polyacrylonitrile, a cellulosic or its derivatives and a polyimidazole.

7. The method of claims 1, 2 or 3 wherein said second fiber is an elastomer selected from the group consisting of spandex and rubber.

8. The method of claims 2 or 3 wherein a double helix of said first fiber is formed around said second fiber while said second fiber is substantially elongated and thus under tension.

9. The fabric produced by the methods of claims 1, 2 or 3.

10. A method of forming a cast from the fabric of claim 9 comprising slipping a tube of said fabric over an extremity requiring the cast and applying sufficient heat and immobilizing agent to the fabric to substantially selectively harden the same.

11. The method of claims 1, 2 or 3 wherein said immobilizing agent comprises an aqueous starch solution.

12. The method of claims 1, 2 or 3 wherein said immobilizing agent comprises an aqueous gelatin solution.

13. The method of claims 1, 2 or 3 wherein said immobilizing agent comprises α-cyanoacrylic acid esters of the following structure:

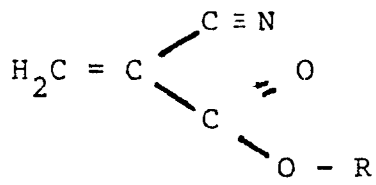

wherein R is a member selected from the group consisting of $CH_3$-, $CH_3CH_2$-, $CH_3CH_2CH_2$-, $CH_3CH_3CH$- and aliphatic, aromatic or mixed aliphatic|aromatic substituents where $C \leq 17$.

14. The method of claim 13 wherein said fabric contains a sensitizer.

15. The method of claim 14 wherein said sensitizer is an anionic or cationic detergent or surfactant.

16. The method of claim 13 wherein polymerization of said α-cyanoacrylic acid ester is enhanced by the application of an alkaline generating material.

17. The method of claim 16 wherein said alkaline generating material is an aryl alkyl amine.

18. The method of claim 1, 2 or 3 wherein said immobilizing agent comprises an aromatic diisocyanate.

19. The method of claim 18 wherein said diisocyanate is 4,4-methyl diphenyl diisocyanate.

20. The method of claims 1,2 or 3 wherein said immobilizing agent is a sulfonated polyester.